# EUROPÄISCHE PATENTSCHRIFT

(11) **EP 3 430 248 B1**
(45) Veröffentlichungstag und Bekanntmachung des Hinweises auf die Patenterteilung: **22.01.2020**
(21) Anmeldenummer: 17702642.4
(22) Anmeldetag: 02.02.2017
(51) Int. Cl.: F01N 3/021, F01N 3/023, F01N 3/025, F01N 3/027, F01N 3/20, F01N 9/00, F01N 11/00, F01N 13/00, F02M 26/00, F02D 41/00, F02D 41/02, F02D 41/14, F02D 41/24, G01N 33/00

(54) **VERFAHREN ZUM ANPASSEN DER KENNLINIE EINES STICKOXIDSENSORS IN EINER BRENNKRAFTMASCHINE**
METHOD FOR ADAPTING CHARACTERISTIC CURVE OF NITROGEN OXIDES SENSOR IN COMBUSTION ENGINE
PROCÉDÉ D'ADAPTATION DE LA COURBE CARACTÉRISTIQUE D'UN CAPTEUR DES OXYDES D'AZOTES DANS UN MOTEUR À COMBUSTION

(30) Priorität: 16.03.2016 DE 102016204324
(43) Veröffentlichungstag der Anmeldung: 23.01.2019
(73) Patentinhaber: Vitesco Technologies GmbH, 30165 Hannover (DE)
(72) Erfinder: ZHANG, Hong, 93105 Tegernheim (DE)
(74) Vertreter: Waldmann, Georg Alexander
(86) Internationale Anmeldenummer: PCT/EP2017/052306
(87) Internationale Veröffentlichungsnummer: WO 2017/157568

(56) Entgegenhaltungen:
- EP-A1- 2 904 386
- WO-A1-2014/187516
- DE-A1-102010 027 984
- JP-A- 2015 001 206
- US-A1- 2011 252 767

## Beschreibung

Die vorliegende Erfindung betrifft ein Verfahren zum Anpassen der Kennlinie eines Stickoxidsensors in einer Brennkraftmaschine, insbesondere ein Verfahren zum Anpassen der Kennlinie eines stromaufwärts eines SCR-Katalysators angeordneten Stickoxidsensors in einer Brennkraftmaschine mit Abgasrückführung.

Bei Brennkraftmaschinen, insbesondere Diesel-Brennkraftmaschinen, ist bekannt, sogenannte SCR(selektive katalytische Reduktion)-Katalysatoren einzusetzen, die zur Reduktion von Stickoxiden in den Abgasen der Brennkraftmaschine eingesetzt werden. Dabei ist die chemische Reaktion am SCR-Katalysator selektiv, das heißt, dass bevorzugt die Stickoxide (NO, NO₂) reduziert werden, während unerwünschte Nebenreaktionen, wie die Oxidation von Schwefeldioxid zu Schwefeltrioxid, weitgehend unterdrückt werden.

Für die chemische Reaktion wird ein Harnstoff stromaufwärts des SCR-Katalysators in das Abgas eingespritzt, der sich daraufhin zumindest teilweise in Ammoniak zersetzt, welches mit dem Abgas zu Wasser und Stickstoff innerhalb des SCR-Katalysators reagieren kann. Zur Steuerung der einzuspritzenden Harnstoffmenge werden beispielsweise Stickoxidsensoren und Ammoniaksensoren eingesetzt, um die jeweiligen Anteile im Auslasstrakt der Brennkraftmaschine zu messen und daraufhin die korrekte Menge des einzuspritzenden Harnstoffs zu steuern.

Die EP 2 904 386 A1 betrifft ein Verfahren zur Verarbeitung von Messwerten eines ersten Stickoxid-Sensors, angeordnet stromauf eines Sickoxidreduktionskatalysators im Abgasstrang eines Kraftfahrzeugs, und von Messwerten eines zweiten Stickoxid-Sensors, angeordnet stromab des Stickoxidreduktionskatalysators im Abgasstrang des Kraftfahrzeugs, bei welchem wenigstens annähernd zeitgleich Messwerte des ersten und zweiten Stickoxid-Sensors erfasst und miteinander verglichen werden. Dabei wird eine Empfindlichkeit des ersten Stickoxid-Sensors und/oder des zweiten Stickoxid-Sensors in Abhängigkeit vom Ergebnis des Vergleichs verändert.

Aus der DE 10 2010 027 984 A1 ist ein Verfahren zum Betreiben einer Abgasanlage einer Brennkraftmaschine bekannt, bei dem mindestens ein Parameter des in einem Abgaskanal strömenden Abgases von einer Abgassonde erfasst wird, wobei während eines Betriebszustandes des Brennkraftmaschine, in dem eine Einspritzung und Verbrennung von Kraftstoff nicht stattfindet, dem Abgaskanal stromaufwärts von der Abgassonde mittels einer der Abgasanlage zugeordneten Frischluftversorgung Frischluft zugeführt wird, und währenddessen und/oder danach die Abgassonde abgeglichen wird.

Die WO 2014/187516 A1 betrifft ein Verfahren zum Betreiben einer Antriebseinrichtung mit einer Brennkraftmaschine und einer Abgasreinigungseinrichtung. Mittels eines ersten Stickoxidsensors stromaufwärts eines Katalysatorelements wird eine erste Stickoxidkonzentrationsgröße und ... eines zweiten Stickoxidsensors stromabwärts des Katalysatorelements wird eine zweite Stickoxidkonzentrationsgröße bestimmt, wobei eine Stickoxidkonzentration aus der Differenz zwischen der ersten und zweiten Stickoxidkonzentrationsgröße ermittelt wird. Dabei ist vorgesehen, dass ein Kalibrieren des ersten Stickoxidsensors und des zweiten Stickoxidsensors bei einer Abgastemperatur vorgenommen wird, für welche die Umwandlungsrate kleiner als eine bestimmte Umwandlungsrate ist.

Aus der JP 2015001206 A ist ein Verfahren und eine Vorrichtung für die Diagnose eines NOX-Sensors bekannt. Hierzu wird während einer DPF Regeneration stromauf eines SCR-Katalysators die Harnstoffzufuhr stromauf des SCR-Katalysators unterbrochen. Ein stromauf des SCR-Katalysators angeordneter NOX-Sensor erfasst die NOX-Emissionen stromab des DPF und eine Steuereinheit bewertet eine Abweichung zu dem NOX-ensor stromab des SCR-Katalysators und bestimmt somit einen Fehler des stromabwärtigen NOX-Sensors.

Aus der US 2011/0252767 A1 ist eine Vorrichtung und ein Verfahren zur Diagnose eines NOx-Sensors bekannt.

Der vorliegenden Erfindung liegt die Aufgabe zugrunde, ein Verfahren bereitzustellen, mit dem eine Kennlinienabweichung eines Stickoxidsensors über die Laufzeit reduziert werden kann.

Der vorliegenden Erfindung liegt der Gedanke zugrunde, die Kennlinie eines stromaufwärts eines SCR-Katalysators angeordneten ersten Stickoxidsensors einer Brennkraftmaschine mit Abgasrückführung mittels der ermittelten Stickoxidwerte eines stromabwärts des SCR-Katalysators zweiten Stickoxidsensors dann zu korrigieren, wenn sich ein stromaufwärts des SCR-Katalysators angeordneter Partikelfilter in einer Regenerationsphase befindet. Während der Regenerationsphase des Partikelfilter ist üblicherweise die Stickoxidemission aufgrund der erhöhten Abgasrückführungsrate verringert, wobei zusätzlich die Harnstoffzufuhr durch eine stromaufwärts des SCR-Katalysators angeordneten Harnstoffeinspritzvorrichtung unterbrochen ist. Insbesondere würde bei der erhöhten Abgastemperatur das durch Zersetzung des Harnstoffs gebildete Ammoniak oxidiert werden, bevor es mit den Stickoxiden im SCR-Katalysator reagieren könnte.

Gemäß der vorliegenden Erfindung ist ein Verfahren zum Anpassen der Kennlinie eines stromaufwärts eines SCR-Katalysators angeordneten ersten Stickoxidsensors einer Brennkraftmaschine mit Abgasrückführung offenbart, die den SCR-Katalysator, den stromaufwärts des SCR-Katalysators angeordneten ersten Stickoxidsensor, einen stromabwärts des SCR-Katalysators angeordneten zweiten Stickoxidsensor, einen stromaufwärts des SCR-Katalysators angeordneten Partikelfilter und eine stromaufwärts des SCR-Katalysators angeordnete Harnstoffeinspritzvorrichtung aufweist. Das erfindungsgemäße Verfahren umfasst ein Bestimmen, das sich der Partikelfilter in einer Regenerationsphase befindet, ein Erhöhen der Abgasrückführungsrate während der Regenerationsphase des Partikelfilters, ein Unterbrechen der Harnstoffzufuhr durch die Harnstoffeinspritzungsvorrichtung während der Regenerationsphase des Partikelfilters, ein Ermitteln von ersten Stickoxidwerten aus den vom ersten Stickoxidsensor während der Regenerationsphase des Partikelfilters erzeugten ersten Stickoxidsignalen, ein Bestimmen, das die ersten Stickoxidwerte innerhalb eines ersten Stickoxidintervalls liegen, ein Ermitteln von zweiten Stickoxidwerten aus den vom zweiten Stickoxidsensor während der Regenerationsphase des Partikelfilters erzeugten zweiten Stickoxidsignalen, ein Bestimmen, das die zweiten Stickoxidwerte innerhalb eines zweiten Stickoxidintervalls liegen, und ein Anpassen der Kennlinie des ersten Stickoxidsensors mittels der zweiten Stickoxidwerte.

Durch das Bestimmen, dass die ersten Stickoxidwerte innerhalb eines ersten Stickoxidintervalls liegen, und durch das Bestimmen, dass die zweiten Stickoxidwerte innerhalb eines zweiten Stickoxidintervalls liegen, wird überprüft, ob die jeweiligen aus den jeweiligen Signalen des ersten und zweiten Stickoxidsensors ermittelten Stickoxidwerte im Wesentlichen stabil sind. Anstelle der Überprüfung der Stabilität der Stickoxidwerte können aufgrund der im Wesentlichen linearen Verläufe der Kennlinien der Stickoxidsensoren auch direkt die jeweiligen Stickoxidsignale überprüft werden, ob diese innerhalb einem Intervall sind bzw. ob diese stabil sind.

In einer bevorzugten Ausgestaltung beschreibt das erste Stickoxidintervall ein Intervall, das sich um einen aus den ersten Stickoxidwerten ermittelten ersten Mittelwert mit ±10 %, insbesondere ±5 %, erstreckt. Ferner beschreibt dabei das zweite Stickoxidintervall ein Intervall, das sich um einen aus den zweiten Stickoxidwerten ermittelten zweiten Mittelwert mit ±10 %, insbesondere ±5 %, erstreckt.

In einer weiteren bevorzugten Ausführungsform weist die Brennkraftmaschine ferner einen stromabwärts des SCR-Katalysators angeordneten Ammoniaksensor auf. In dieser bevorzugten Ausgestaltung umfasst das erfindungsgemäße Verfahren ferner ein Erzeugen eines Ammoniaksignals mittels des Ammoniaksensors, ein Ermitteln eines Ammoniakwerts aus den vom Ammoniaksensor erzeugten Ammoniaksignal und ein Bestimmten, dass der ermittelte Ammoniakwert kleiner als ein vorbestimmter Ammoniakschwellenwert ist. Nur wenn bestimmt wird, dass der ermittelte Ammoniakwert kleiner als der vorbestimmte Ammoniakschwellenwert ist, wird die Anpassung der Kennlinie des ersten Stickoxidsensors gemäß dem vorliegenden Verfahren durchgeführt. Im Umkehrschluss bedeutet dies, dass bei dem Bestimmen, dass der ermittelte Ammoniakwert größer als der vorbestimmte Ammoniakschwellenwert ist, die Kennlinienadaption gemäß der vorliegenden Erfindung nicht durchgeführt werden darf.

Gemäß einer bevorzugten Ausgestaltung beträgt der vorbestimmte Ammoniakschwellenwert ungefähr 5 ppm, insbesondere ungefähr 1 ppm.

Gemäß einer weiteren vorteilhaften Ausgestaltung weist das erfindungsgemäße Verfahren ferner ein Ermitteln eines absoluten Änderungsgradienten der Ammoniaksignale und ein Bestimmen auf, dass der absolute Änderungsgradient der Ammoniaksignale unterhalb eines vorbestimmten Ammoniakänderungsschwellenwert liegt.

Im Rahmen der vorliegenden Offenbarung beschreibt ein absoluter Änderungsgradient eine zeitliche Änderung der innerhalb eines vorbestimmten Zeitintervalls kurzzeitig hintereinander erfassten Signale. Insbesondere kann der Änderungsgradient ein Indiz für stabile Werte bzw. Signale sein.

Vorteilhafterweise wird bei der Anpassung der Kennlinie des ersten Stickoxidsensors die Zeitverzögerung zwischen dem Signal des ersten Stickoxidsensor und dem Signal des zweiten Stickoxidsensors berücksichtigt. In Abhängigkeit des Strömungsverhaltens des Abgases, beispielsweise Strömungsgeschwindigkeit und/oder Strömungsweglänge zwischen dem ersten Stickoxidsensor und dem zweiten Stickoxidsensor, sind die eine Abgasprobe charakterisierenden Signale zwischen dem ersten Stickoxidsensor und dem zweiten Stickoxidsensor zeitlich versetzt. Vorzugsweise wird diesem zeitlichen Versatz Rechnung getragen und in der Anpassung berücksichtigt.

Gemäß einem weiteren Aspekt der vorliegenden Erfindung ist ein Auslasstrakt für eine Brennkraftmaschine mit Abgasrückführung offenbart, der einen SCR-Katalysator, einen stromaufwärts des SCR-Katalysators angeordneten Partikelfilter, insbesondere Diesel-Partikelfilter, einen stromaufwärts des SCR-Katalysators angeordneten ersten Stickoxidsensor, der dazu ausgebildet ist, ein den Stickoxidwert anzeigendes erstes Stickoxidsignal zu erzeugen, eine stromaufwärts des SCR-Katalysators angeordnete Harnstoffeinspritzvorrichtung, die dazu ausgebildet ist, eine vorbestimmte Harnstoffmenge einzuspritzen, einen stromabwärts des SCR-Katalysators angeordneten zweiten Stickoxidsensor, der dazu ausgebildet ist, ein den Stickoxidwert anzeigendes zweites Stickoxidsignal zu erzeugen, einen stromabwärts des SCR-Sensors angeordneten Ammoniaksensor, der dazu ausgebildet ist, ein den Ammoniakwert anzeigendes Ammoniaksignal zu erzeugen, und eine Steuereinheit aufweist, die dazu ausgebildet ist, das erste Stickoxidsignal, das zweite Stickoxidsignal und das Ammoniaksignal zu empfangen und ein Verfahren gemäß der vorliegenden Offenbarung auszuführen.

Weitere Merkmale und Aufgaben der vorliegenden Erfindung werden dem Fachmann durch Betrachten der beiliegenden Zeichnungen ersichtlich, in denen:
- Fig. 1: einen Teil eines beispielhaft offenbarten Auslasstrakts einer Brennkraftmaschine mit Abgasrückführung zeigt und
- Fig. 2: ein Flussdiagramm eines beispielhaften Verfahrens zur Anpassung der Kennlinie eines Stickoxidsensors gemäß der vorliegenden Offenbarung darstellt.

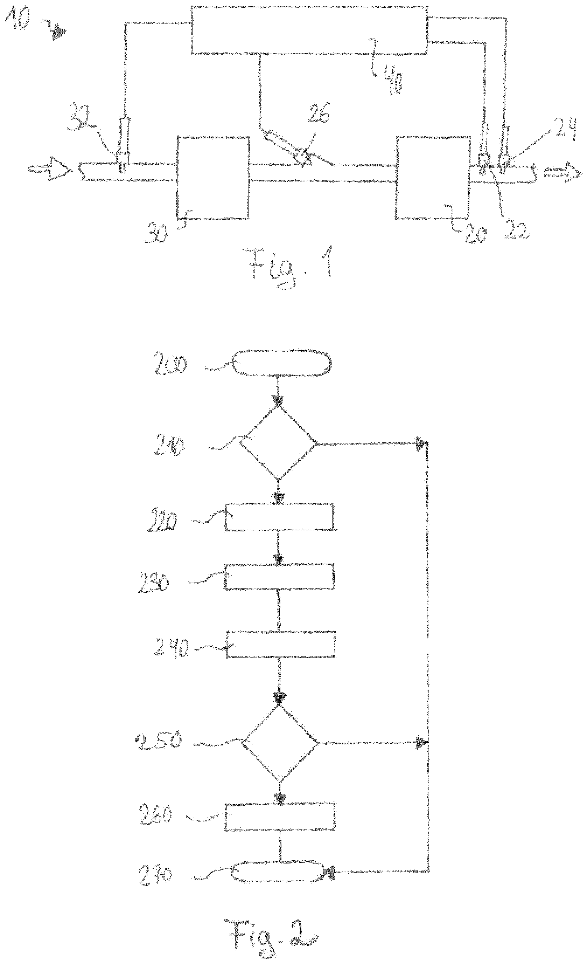

Die Figur 1 zeigt schematisch einen Teil eines Auslasstrakts 10 einer Brennkraftmaschine (nicht näher dargestellt). Der Auslasstrakt 10 weist einen SCR-Katalysator 20 auf, der dazu ausgebildet ist, eine chemische Reaktion durchzuführen, damit die Stickoxide im Abgas reduziert werden können. Stromaufwärts des SCR-Katalysators 20 ist ein Partikelfilter 30, beispielsweise ein Dieselpartikelfilter, angeordnet. In einer bevorzugten Ausgestaltung sind der SCR-Katalysator 20 und der Partikelfilter 30 in einer Bauteileinheit integriert. Stromabwärts des SCR-Katalysators 20 sind ein Stickoxidsensor 22 und ein Ammoniaksensor 24 angeordnet, die dazu ausgebildet sind, entsprechende Signale zu erzeugen. Insbesondere ist der Stickoxidsensor 22 dazu ausgebildet, ein einen Stickoxidwert anzeigendes Stickoxidsignal zu erzeugen. In ähnlicher Weise ist der Ammoniaksensor 24 dazu ausgebildet, ein einen Ammoniakwert anzeigendes Ammoniaksignal zu erzeugen.

Stromaufwärts des SCR-Katalysators 20 ist eine Harnstoffeinspritzvorrichtung 26 angeordnet, die dazu ausgebildet ist, zu vorbestimmten Zeitpunkten eine vorbestimmte Harnstoffmenge einzuspritzen. Die Harnstofflösung ist dazu ausgebildet, vom Abgas derart zersetzt zu werden, dass zumindest teilweise Ammoniak entsteht, welches in dem SCR-Katalysator 20 chemisch reagieren kann und somit die Stickoxide im Abgas reduzieren kann.

Gemäß der in der Fig. 1 dargestellten beispielhaften Ausgestaltung des Auslasstrakts 10 ist ferner ein weiterer Stickoxidsensor 32 stromaufwärts des Partikelfilters 20 vorgesehen, der dazu ausgebildet ist, ein einen Stickoxidewert anzeigendes weiteres Stickoxidsignal zu erzeugen.

Eine Steuereinheit 40, die beispielsweise Bestandteil der Steuerung der Brennkraftmaschine sein kann, ist mit dem Stickoxidsensor 22, dem Ammoniaksensor 24, der Harnstoffeinspritzvorrichtung 26 und dem weiteren Stickoxidsensor 32 verbunden und dazu ausgebildet, Signale von diesen Vorrichtungen zu empfangen bzw. an diese zur Steuerung derselben zu senden.

Beispielsweise ist die Steuerung 40 dazu ausgebildet, ein Verfahren gemäß der Fig. 2 auszuführen. Die Fig. 2 zeigt ein beispielhaftes Flussdiagramm eines Verfahrens zum Anpassen der Kennlinie eines Stickoxidsensors einer Brennkraftmaschine mit Abgasrückführung.

Das Verfahren startet am Schritt 200 und es wird am Schritt 210 bestimmt, dass sich der Partikelfilter 30 in einer Regenerationsphase befindet. Beispielsweise ist eine Regenerationsphase des Partikelfilters dann notwendig, wenn die Beladung des Partikelfilters einen vorbestimmten Schwellenwert, beispielsweise mehr als 90% der maximalen Beladung, überschreitet. Wenn am Schritt 210 bestimmt wird, dass sich der Partikelfilter 30 nicht in einer Regenerationsphase befindet, gelangt das Verfahren zum Schritt 270 und endet dort.

Wenn am Schritt 210 bestimmt wird, dass sich der Partikelfilter 30 in einer Regenerationsphase befindet, wird am Schritt 220 die Abgasrückführungsrate erhöht. Am folgenden Schritt 230 wird die Harnstoffzufuhr durch die Harnstoffeinspritzvorrichtung 26 unterbrochen. In einem anschließenden Schritt 240 werden aus den vom ersten Stickoxidsensor 32 ermittelten ersten Stickoxidsignalen erste Stickoxidwerte ermittelt. Gleichzeitig werden am Schritt 240 aus den vom zweiten Stickoxidsensor 22 ermittelten zweiten Stickoxidsignalen zweite Stickoxidwerte ermittelt.

An einem darauffolgenden Schritt 250 wird überprüft, ob die ersten bzw. zweiten Stickoxidwerte innerhalb eines ersten Stickoxidintervalls bzw. innerhalb eines zweiten Stickoxidintervalls liegen. Das heißt, dass bestimmt wird, ob die ersten bzw. zweiten Stickoxidsignale im Wesentlichen stabil sind. Wenn am Schritt 250 bestimmt wird, dass die ersten Stickoxidsignale nicht innerhalb des ersten Stickoxidintervalls liegen und/oder die zweiten Stickoxidsignale nicht innerhalb des zweiten Stickoxidintervalls liegen, gelangt das Verfahren zum Schritt 270, an dem es beendet wird.

Wenn jedoch am Schritt 250 bestimmt wird, dass sowohl die ersten Stickoxidsignale innerhalb des ersten Stickoxidintervalls liegen als auch die zweiten Stickoxidsignale innerhalb des zweiten Stickoxidintervalls liegen, erfolgt am Schritt 260 eine Anpassung der Kennlinie des ersten Stickoxidsensors 32 mittels der Stickoxidwerte des zweiten Stickoxidsensors 22.

Das Verfahren gemäß der Fig. 2 kann dahingehend erweitert werden, dass aus dem vom Ammoniaksensor 24 erzeugten Ammoniaksignal ein Ammoniakwert ermittelt wird. Daraufhin kann bestimmt werden, ob der ermittelte Ammoniakwert kleiner als ein vorbestimmter Ammoniakschwellenwert ist, beispielsweise ungefähr 5 ppm, insbesondere ungefähr 1 ppm.

Insbesondere ist es vorteilhaft, die Anpassung am Schritt 260 erst dann vorzunehmen, wenn bestimmt wird, dass die ermittelte Ammoniakmenge kleiner als der vorbestimmte Ammoniakschwellenwert ist. Das bedeutet im Umkehrschluss, dass eine solche Anpassung nicht stattfindet, wenn der Ammoniakwert größer als der vorbestimmte Ammoniakschwellenwert ist.

In ähnlicher Weise entfällt die oben dargelegte Anpassung der Kennlinie des ersten Stickoxidsensors 32 wenn bestimmt wird, dass zusätzlich oder alternativ zum Überschreiten des Ammoniakschwellenwerts ein absoluter Änderungsgradient der Ammoniaksignale des Ammoniaksensors einen vorbestimmten Ammoniakänderungsschwellenwert überschreitet.

## Patentansprüche

1. Verfahren zum Anpassen der Kennlinie eines stromaufwärts eines SCR-Katalysators (20) angeordneten ersten Stickoxidsensors (32) einer Brennkraftmaschine mit Abgasrückführung, die den SCR-Katalysator (20), den stromaufwärts des SCR-Katalysators (20) angeordneten ersten Stickoxidsensor (32), einen stromabwärts des SCR-Katalysators (20) angeordneten zweiten Stickoxidsensor (22), einen stromaufwärts des SCR-Katalysators (20) angeordneten Partikelfilter (30) und eine stromaufwärts des SCR-Katalysators (20) angeordnete Harnstoffeinspritzvorrichtung (26) aufweist, **dadurch gekennzeichnet, dass** das Verfahren umfasst:
- Bestimmen (210), dass sich der Partikelfilter (30) in einer Regenerationsphase befindet,
- Erhöhen (220) der Abgasrückführungsrate während der Regenerationsphase des Partikelfilters (30),
- Unterbrechen (230) der Harnstoffzufuhr durch die Harnstoffeinspritzvorrichtung (26) während der Regenerationsphase des Partikelfilters (30),
- Ermitteln (240) von ersten Stickoxidwerten aus vom ersten Stickoxidsensor (32) während der Regenerationsphase des Partikelfilters (30) erzeugten ersten Stickoxidsignalen und Bestimmen, dass die ersten Stickoxidwerte innerhalb eines ersten Stickoxidintervalls liegen,
- Ermitteln (250) von zweiten Stickoxidwerten aus vom zweiten Stickoxidsensor (22) während der Regenerationsphase des Partikelfilters (30) erzeugten zweiten Stickoxidsignalen und Bestimmen, dass die zweiten Stickoxidwerte innerhalb eines zweiten Stickoxidintervalls liegen, und
- Anpassen (260) der Kennlinie des ersten Stickoxidsensors (32) mittels der zweiten Stickoxidwerte.

2. Verfahren nach Anspruch 1, wobei
das erste Stickoxidintervall ein Intervall beschreibt, das sich um einen aus den ersten Stickoxidwerten ermittelten ersten Mittelwert mit ±10 %, insbesondere ±5 %, erstreckt, und
das zweite Stickoxidintervall ein Intervall beschreibt, das sich um einen aus den zweiten Stickoxidwerten ermittelten zweiten Mittelwert mit ±10 %, insbesondere ±5 %, erstreckt.

3. Verfahren nach einem der vorhergehenden Ansprüche, wobei die Brennkraftmaschine ferner einen stromabwärts des SCR-Katalysators (20) angeordneten Ammoniaksensor (24) aufweist, ferner umfassend:
- Erzeugen eines Ammoniaksignals mittels des Ammoniaksensors (24),
- Ermitteln eines Ammoniakwerts aus dem dem Ammoniaksignal, und
- Bestimmen, dass der ermittelte Ammoniakwert kleiner als ein vorbestimmter Ammoniakschwellenwert ist.

4. Verfahren nach Anspruch 3, wobei der vorbestimmte Ammoniakschwellenwert ungefähr 5 ppm, insbesondere ungefähr 1 ppm beträgt.

5. Verfahren nach einem der Ansprüche 3 oder 4, ferner mit:
- Ermitteln eines absoluten Änderungsgradienten der Ammoniaksignale, und
- Bestimmen, dass der absolute Änderungsgradient der Ammoniaksignale unterhalb eines vorbestimmten Ammoniakänderungsschwellenwerts liegt.

6. Verfahren nach einem der vorhergehenden Ansprüche, wobei zwischen dem Erzeugen der ersten Stickoxidsignale mittels des ersten Stickoxidsensors (32) und dem Erzeugen der zweiten Stickoxidsignale mittels des zweiten Stickoxidsensors (22) ein vorbestimmter Zeitunterschied befindet und bei der Anpassung der Kennlinie des erste Stickoxidsensors (32) dieser vorbestimmte Zeitunterschied in den zweiten Stickoxidwerten berücksichtigt ist.

7. Auslasstrakt (10) für eine Brennkraftmaschine mit Abgasrückführung, mit:
- einem SCR-Katalysator (20),
- einem stromaufwärts des SCR-Katalysators (20) angeordneten Partikelfilter (30);
- einem stromaufwärts des SCR-Katalysators (20) angeordneten ersten Stickoxidsensor (32), der dazu ausgebildet ist, ein den Stickoxidwert stromaufwärts des SCR-Katalysators (20) anzeigendes erstes Stickoxidsignal zu erzeugen,
- einer stromaufwärts des SCR-Katalysators (20) angeordneten Harnstoffeinspritzvorrichtung (26), die dazu ausgebildet ist, eine vorbestimmte Harnstoffmenge einzuspritzen,
- einem stromabwärts des SCR-Katalysators (20) angeordneten zweiten Stickoxidsensor (22), der dazu ausgebildet ist, ein den Stickoxidwert stromabwärts des SCR-Katalysators (20) anzeigendes zweites Stickoxidsignal zu erzeugen,
- einem stromabwärts des SCR-Katalysators (20) angeordneten Ammoniaksensor (24), der dazu ausgebildet ist, ein den Ammoniakwert stromabwärts des SCR-Katalysators (20) anzeigendes Ammoniaksignal zu erzeugen, und
- einer Steuereinheit (40), die dazu ausgebildet ist, das erste Stickstoffoxidsignal, das zweite Stickoxidsignal und das Ammoniaksignal zu empfangen und **dadurch gekennzeichnet** ein Verfahren nach einem der vorhergehenden Ansprüche auszuführen.

## Claims

1. Method for adapting the characteristic curve of a first nitrogen oxide sensor (32), arranged upstream of an SCR catalytic converter (20), of an internal combustion engine with exhaust gas recirculation, which has the SCR catalytic converter (20), the first nitrogen oxide sensor (32) arranged upstream of the SCR catalytic converter (20), a second nitrogen oxide sensor (22) arranged downstream of the SCR catalytic converter (20), a particle filter (30) arranged upstream of the SCR catalytic converter (20), and a urea injection device (26) arranged upstream of the SCR catalytic converter (20), **characterized in that** the method comprises:
- determining (210) that the particle filter (30) is in a regeneration phase,
- increasing (220) the exhaust gas recirculation rate during the regeneration phase of the particle filter (30),
- interrupting (230) the supply of urea by the urea injection device (26) during the regeneration phase of the particle filter (30),
- acquiring (240) first nitrogen oxide values from first nitrogen oxide signals generated by the first nitrogen oxide sensor (32) during the regeneration phase of the particle filter (30), and determining that the first nitrogen oxide values are within a first nitrogen oxide interval,
- acquiring (250) second nitrogen oxide values from second nitrogen oxide signals generated by the second nitrogen oxide sensor (22) during the regeneration phase of the particle filter (30), and determining that the second nitrogen oxide values are within a second nitrogen oxide interval,
- adapting (260) the characteristic curve of the first nitrogen oxide sensor (32) by means of the second nitrogen oxide values.

2. Method according to Claim 1, wherein
the first nitrogen oxide interval describes an interval which extends about a first mean value, acquired from the first nitrogen oxide values, with ±10 %, in particular ±5%, and
the second nitrogen oxide interval describes an interval which extends about a second mean value, acquired from the second nitrogen oxide values, with ±10 %, in particular ±5%.

3. Method according to one of the preceding claims, wherein the internal combustion engine also has an ammonia sensor (24) arranged downstream of the SCR catalytic converter (20), also comprising:
- generating an ammonia signal by means of the ammonia sensor (24),
- acquiring an ammonia value from the ammonia signal, and
- determining that the acquired ammonia value is lower than a predetermined ammonia threshold value.

4. Method according to Claim 3, wherein the predetermined ammonia threshold value is approximately 5 ppm, in particular approximately 1 ppm.

5. Method according to either of Claims 3 and 4, also comprising:
- acquiring an absolute change gradient of the ammonia signals, and
- determining that the absolute change gradient of the ammonia signals is below a predetermined ammonia change threshold value.

6. Method according to one of the preceding claims, wherein there is a predetermined time difference between the generation of the first nitrogen oxide signals by means of the first nitrogen oxide sensor (32) and the generation of the second nitrogen oxide signals by means of the second nitrogen oxide sensor (22), and during the adaptation of the characteristic curve of the first nitrogen oxide sensor (32) this predetermined time difference is taken into account in the second nitrogen oxide values.

7. Exhaust section (10) for an internal combustion engine with exhaust gas recirculation, comprising:
- an SCR catalytic converter (20);
- a particle filter (30) arranged upstream of the SCR catalytic converter (20);
- a first nitrogen oxide sensor (32) which is arranged upstream of the SCR catalytic converter (20) and is designed to generate a first nitrogen oxide signal which indicates the nitrogen oxide value upstream of the SCR catalytic converter (20),
- a urea injection device (26) which is arranged upstream of the SCR catalytic converter (20) and is designed to inject a predetermined quantity of urea,
- a second nitrogen oxide sensor (22) which is arranged downstream of the SCR catalytic converter (20) and is designed to generate a second nitrogen oxide signal which indicates the nitrogen oxide value downstream of the SCR catalytic converter (20),
- an ammonia sensor (24) which is arranged downstream of the SCR catalytic converter (20) and is designed to generate an ammonia signal which indicates the ammonia value downstream of the SCR catalytic converter (20), and
- a control unit (40) which is designed to receive the first nitrogen oxide signal, the second nitrogen oxide signal and the ammonia signal and characterized to execute a method according to one of the preceding claims.

## Revendications

1. Procédé d'adaptation de la caractéristique d'un premier capteur d'oxyde d'azote (32), disposé en amont d'un catalyseur SCR (20), d'un moteur à combustion interne à recirculation de gaz d'échappement qui comporte le catalyseur SCR (20), le premier capteur d'oxyde d'azote (32) disposé en amont du catalyseur SCR (20), un deuxième capteur d'oxyde d'azote (22) disposé en aval du catalyseur SCR (20), un filtre à particules (30) disposé en amont du catalyseur SCR (20) et un dispositif d'injection d'urée (26) disposé en amont du catalyseur SCR (20), **caractérisé en ce que** le procédé comprend les étapes suivantes :
- déterminer (210) si le filtre à particules (30) est dans une phase de régénération,
- augmenter (220) le taux de recirculation de gaz d'échappement pendant la phase de régénération du filtre à particules (30),
- interrompre (230) l'alimentation en urée par le dispositif d'injection d'urée (26) pendant la phase de régénération du filtre à particules (30),
- déterminer (240) des premières valeurs d'oxyde d'azote à partir de premiers signaux d'oxyde d'azote générés par le premier capteur d'oxyde d'azote (32) pendant la phase de régénération du filtre à particules (30) et déterminer si les premières valeurs d'oxyde d'azote se situent dans un premier intervalle d'oxyde d'azote,
- déterminer (250) des deuxièmes valeurs d'oxyde d'azote à partir de deuxièmes signaux d'oxyde d'azote générés par le deuxième capteur d'oxyde d'azote (22) pendant la phase de régénération du filtre à particules (30) et déterminer si les deuxièmes valeurs d'oxyde d'azote se situent dans un deuxième intervalle d'oxyde d'azote, et
- adapter (260) la caractéristique du premier capteur d'oxyde d'azote (32) à l'aide des deuxièmes valeurs d'oxyde d'azote.

2. Procédé selon la revendication 1,
le premier intervalle d'oxyde d'azote décrivant un intervalle qui s'étend de ± 10 %, en particulier de ± 5 %, autour d'une première valeur moyenne déterminée à partir des premières valeurs d'oxyde d'azote, et
le deuxième intervalle d'oxyde d'azote décrivant un intervalle qui s'étend de ± 10 %, en particulier de ± 5 %, autour d'une deuxième valeur moyenne déterminée à partir des deuxièmes valeurs d'oxyde d'azote.

3. Procédé selon l'une des revendications précédente, le moteur à combustion interne comprenant en outre un capteur d'ammoniac (24) disposé en aval du catalyseur SCR (20), le procédé comprenant en outre les étapes suivantes :
- générer un signal d'ammoniac au moyen du capteur d'ammoniac (24),
- déterminer une valeur d'ammoniac à partir du signal d'ammoniac, et
- déterminer si la valeur d'ammoniac déterminée est inférieure à un seuil d'ammoniac prédéterminé.

4. Procédé selon la revendication 3, le seuil d'ammoniac prédéterminé étant d'environ 5 ppm, notamment d'environ 1 ppm.

5. Procédé selon l'une des revendications 3 et 4, le procédé comprenant en outre les étapes suivantes :
- déterminer un gradient absolu de variation des signaux d'ammoniac, et
- déterminer si le gradient absolu de variation des signaux d'ammoniac est inférieur à un seuil de variation d'ammoniac prédéterminé.

6. Procédé selon l'une des revendications précédentes, une différence de temps prédéterminée étant présente entre l'étape de génération des premiers signaux d'oxyde d'azote au moyen du premier capteur d'oxyde d'azote (32) et l'étape de génération des deuxièmes signaux d'oxyde d'azote au moyen du deuxième capteur d'oxyde d'azote (22) et, lors de l'adaptation de la caractéristique du premier capteur d'oxyde d'azote (32), cette différence de temps prédéterminée étant prise en compte dans les deuxièmes valeurs d'oxyde d'azote.

7. Tube d'échappement (10) destiné à un moteur à combustion interne à recirculation de gaz d'échappement, le tube d'échappement comportant :
- un catalyseur SCR (20),
- un filtre à particules (30) disposé en amont du catalyseur SCR (20),
- un premier capteur d'oxyde d'azote (32) disposé en amont du catalyseur SCR (20) et conçu pour générer un premier signal d'oxyde d'azote indiquant la valeur d'oxyde d'azote en amont du catalyseur SCR (20),
- un dispositif d'injection d'urée (26) disposé en amont du catalyseur SCR (20) et conçu pour injecter une quantité d'urée prédéterminée,
- un deuxième capteur d'oxyde d'azote (22) disposé en aval du catalyseur SCR (20) et conçu pour générer un deuxième signal d'oxyde d'azote indiquant la valeur d'oxyde d'azote en aval du catalyseur SCR (20),
- un capteur d'ammoniac (24) disposé en aval du catalyseur SCR (20) et conçu pour générer un signal d'ammoniac indiquant la valeur d'ammoniac en aval du catalyseur SCR (20), et
- une unité de commande (40) conçue pour recevoir le premier signal d'oxyde d'azote, le deuxième signal d'oxyde d'azote et le signal d'ammoniac, et **caractérisée** pour mettre en œuvre d'un procédé selon l'une des revendications précédentes.
